# EUROPEAN PATENT SPECIFICATION

(11) **EP 2 462 240 B1**
(45) Date of publication and mention of the grant of the patent: **05.07.2017**
(21) Application number: 10807057.4
(22) Date of filing: 03.08.2010
(51) Int. Cl.: C07K 14/47, G01N 33/574, G01N 33/68

(54) **A LECTIN ASSAY FOR ASSESSING GLYCOFORMS AS AN EARLY MARKER IN DISEASE**
LEKTIN-ASSAY ZUM TESTEN VON GLYCOFORMEN ALS FRÜHER KRANKHEITSMARKER
DOSAGE DE LECTINE POUR L'ÉVALUATION DE GLYCOFORMES EN TANT QUE MARQUEUR PRÉCOCE D'UNE MALADIE

(30) Priority: 05.08.2009 US 231400 P
(43) Date of publication of application: 13.06.2012
(73) Proprietor: Institute For Hepatitis And Virus Research, Doylestown, PA 18902 (US)
(72) Inventor: ROMANO, Patrick, Robert, Doylestown,PA 18901 (US); MEHTA, Anand, Lansdale,PA 19446 (US); BLOCK, Timothy, M., Doylestown,PA 18901 (US)
(74) Representative: Manaton, Ross Timothy
(86) International application number: PCT/US2010/044307
(87) International publication number: WO 2011/017369

(56) References cited:
- WO-A2-2009/075883
- ADAM JAN ET AL: "Engineering of PA-IIL lectin from Pseudomonas aeruginosa - Unravelling the role of the specificity loop for sugar preference", BMC STRUCTURAL BIOLOGY, BIOMED CENTRAL LTD., LONDON, GB, vol. 7, no. 1, 1 June 2007 (2007-06-01), page 36, XP021029516, ISSN: 1472-6807, DOI: 10.1186/1472-6807-7-36
- JOHAN OLAUSSON ET AL: "Detection of a high affinity binding site in recombinant Aleuria aurantia lectin", GLYCOCONJUGATE JOURNAL, KLUWER ACADEMIC PUBLISHERS, BO, vol. 25, no. 8, 21 May 2008 (2008-05-21), pages 753-762, XP019642423, ISSN: 1573-4986, DOI: 10.1007/S10719-008-9135-7
- COMUNALE M A ET AL: "Proteomic Analysis of Serum Associated Fucosylated Glycoproteins in the Development of Primary Hepatocellular Carcinoma", JOURNAL OF PROTEOME RESEARCH, AMERICAN CHEMICAL SOCIETY, WASHINGTON, DC, US, vol. 5, no. 2, 1 January 2006 (2006-01-01) , pages 308-315, XP002494796, ISSN: 1535-3907, DOI: 10.1021/PR050328X
- AMANO KOH ET AL: "Involvement of tyrosines at fucose-binding sites of Aleuria aurantia lectin: non-equal response to site-directed mutagenesis among five sites", BIOSCIENCE BIOTECHNOLOGY BIOCHEMISTRY, JAPAN SOCIETY FOR BIOSCIENCE, BIOTECHNOLOGY, AND AGROCHEMISTRY, TOKYO, JAPAN, vol. 68, no. 4, 1 April 2004 (2004-04-01), pages 841-847, XP002599542, ISSN: 0916-8451
- PATRICK R. ROMANO ET AL: "Development of recombinant Aleuria aurantia lectins with altered binding specificities to fucosylated glycans", BIOCHEMICAL AND BIOPHYSICAL RESEARCH COMMUNICATIONS, vol. 414, no. 1, 1 October 2011 (2011-10-01), pages 84-89, XP055056001, ISSN: 0006-291X, DOI: 10.1016/j.bbrc.2011.09.027
- ADAM ET AL.: 'Engineering of PA-IIL lectin from Pseudomonas aeruginosa. Unravelling the role of the specificity loop for sugar preference.' BMC STRUCTURAL BIOLOGY vol. 7, no. 36, 2007, pages 1 - 13, XP021029516
- MITCHEL ET AL.: 'Structural basis for oligosaccharide-mediated adhesion of Psuedomonas aeruginosa In the lungs of cystic fibrosis patients.' NATURE STRUCTURAL BIOLOGY vol. 9, no. 12, 2002, pages 918 - 921, XP008152008
- MAROTTE ET AL.: 'Synthesis and binding properties of divalent and trivalent clusters of the Lewis a disaccharide moiety to Pseudomonas aeruginosa lectin PA-II.' ORG. BIOMOL. CHEM. vol. 5, 2007, pages 2953 - 2961, XP008152009
- OLAUSSON ET AL.: 'Detection of a high affinity binding site in recombinant Aleuria aurantia lectin.' GLYCOCONJ J. vol. 25, 2008, pages 753 - 762, XP019642423
- MRAZKOVA. ET AL.: 'Randon mutagenesis as a tool for fine tuning of affinity and specificity of PA- IlL from Pseudomonas aerogenosa.' XIII. SETK?N? BIOCHEMIKU A MOLEKUL?M?CH BIOLOGU 14 April 2009, page 34
- HSU ET AL.: 'A simple strategy for the creation of a recombinant lectin microarray.' MOL. BIOSYST. vol. 4, 2008, pages 654 - 662, XP008152011

## Description

### CROSS-REFERENCE:

The present application claims the benefit of priority of U.S. Provisional Application No. 61/231400, filed August 05, 2009.

### BACKGROUND OF THE INVENTION

Various proteins exist in two or more different isoforms that differ only in their pattern of glycosylation. Such differences, or the relative proportions of the differently glycosylated isoforms, may be indicative of a disease or disorder and thus there is a need for assay systems capable of distinguishing between the differently glycosylated isoforms.

The use of antibodies to distinguish between differently glycosylated isoforms of endogenous proteins is problematic as the success rate in raising antibodies which bind specifically or preferentially to particular isoforms of endogenous glycosylated proteins is relatively low.

Further, determination of the relative concentrations of differentially glycosylated isoforms of an endogenous protein has been shown to be clinically important. Keir et al discusses the abnormal relative abundances of the transferring glycosylated isoforms in patients with carbohydrate deficient glycoprotein syndromes or congenital disorder of glycosylation (Keir et al. Ann. Clin. Biochem. 36: 20-36 (1999). Any protein with post-translational glycosylation can potentially occur in different glycosylation isoforms. Thus clinically relevant proteins may exist in different glycosylated isoforms, including glycosylated markers for cancers and other disease, including alkaline phosphatase, alpha-fetoprotein, human chorionic gonadotropoin, and possibly prion protein (CD230).

Other glycoproteins associated with disorders and considered potential targets for assay development in the present invention include, but not limited to, alpha-1-acid glycoprotein, alpha-1-antitrypsin, haptoglobin, thyroglobulin, prostate specific antigen, HEMPAS erythrocyte band 3 (associated with congenital dyserythropoietic anemia type II), PC-1 plasma-cell membrane glycoprotein, CD41 glycoprotein IIb, CD42b glycocalicin, CD43 leukocyte sialoglycoprotein, CD63 lysosomal-membrane-associated glycoprotein 3, CD66a biliary glycoprotein, CD66f pregnancy specific b1 glycoprotein, CD164 multi-glycosylated core protein 24, and the Cd235 glycophorin family.

Aleuria aurantia lectin (AAL) is a 312 amino acid protein having no carbohydrate chain and containing five binding sites for L-fucose or L-fucose-linked oligosaccharides. The multivalent nature of AAL gives it an unusually high binding affinity (micrormolar) for carbohydrate ligands compared to other lectins. Commercial production of AAL is based on the isolation and purification of lectin by binding to a fucose-starch column. AAL is eluted from the column with 50 mM L-fucose (Fujihashi et al.). However, structural and biochemical analysis has shown that commercial AAL has 3 to 5 of its 5 ligand binding sites occupied with fucose as a result of this manufacturing process (Olausson et al., Amano et al., Fujihashi et al., and Wimmerova et al.). Consequently, AAL produced through these methods lack the specificity and affinity needed for incorporation into a clinically significant diagnostic assay.

Accordingly, there is a need to have an assay that can selectively distinguish two or more glycoprotein isoforms having different glycosylation patterns,, said method comprising contacting a sample containing a glycoprotein expressing two or more glycoforms using a recombinant form of AAL having a high affinity recognition for specific fucosylated oligosaccharides, and directly or indirectly detecting differences in glycoforms as an indicator of disease status.

### BRIEF DESCRIPTION OF THE INVENTION

The present invention incorporates the use of high affinity lectin to detect alterations in target sugar moieties on proteins and provides a means for an automated diagnostic assay in the early detection of diseases. The invention provides a method for distinguishing two or more glycoprotein isoforms according to claim 1 and a method for analysing a serum sample according to claim 6. The invention also provides a method for producing a recombinant AAL, according to claim 9, as well as a recombinant AAL according to claim 15. One embodiment of the present invention is a blood test for individuals with inflammatory disorders, autoimmune disorders, cancer, infections, or other disorders where a change in the glycosylation patterns of specific proteins are used as biomarkers in serum or as biomarkers expressed on the surface of cells. Analysis of the levels of these proteins, either through identification of the glycoform or quantification of the protein levels expressing these glycoforms, provides for a simple blood test for detecting people with disease or people at risk for disease progression. An assay incorporating the use of high affinity lectin as a detector in an assay platform such as, but not limited to, a plate-based or bead-based formats is readily automated for clinical or point-of-care environments.

### BRIEF DESCRIPTION OF THE FIGURES

Figure 1: Overall structure of the AAL monomer with blades 1 through 6. Ellipsoids indicate fucose binding sites 1 through 5 and the corresponding site 6. Three stick modes at sites 1, 2, and 4 show fucose molecules.
Figure 2: Affinity comparisons for IgG0 Ligand Using Nickel NTA Plates.
Figure 3: Indirect ELISA using recombinant AAL proteins in competition with commercial AAL proteins.

### DETAILED DESCRIPTION OF THE INVENTION

The present invention is based on the recent discovery that (1) increased serum levels of fucosylated glycoforms of certain proteins serve as early biomarkers of diseases such as cancer and (2) increased serum levels for agalactosylated glycoform of immunoglobulin G, called alpha-gal IgG0, correlates with diagnosis of liver disease (see Example 1). Accordingly, recombinant or mutant forms of lectin (AAL), linked to any reporter molecule known in the art (e.g. a radiolabel, chromophore or fluorophore), are used as an alpha-gal IgG0 detection molecule, specific for fucosylated proteins in blood. Incorporation into an ELISA or bead based assay systems provides the basis for an automated platform to determine a patients disease status.

One embodiment of the present invention considers the production and isolation of a recombinant wild-type AAL protein in E. coli bacteria and/or yeast. Methods are provided that include the creation and production of mutant AAL proteins altered either by site directed mutagenesis or by random mutagenesis and subsequently selected for mutant AAL proteins. These AAL proteins have a high affinity for the outerarm L-fucopyranosyl linkages, more specifically mutated AAL protein having high affinity for the alpha 1-2 outerarm L-fucopyranosyl linkage, alpha 1-3 outerarm L-fucopyranosyl linkage, or alpha 1-4 outerarm L-fucopyranosyl linkage found in serum protein biomarkers in patient with diseases such as, but not limited to, cancer..

A further embodiment of the present invention incorporates the use of core fucose (alpha 1-6 linked) glycoforms as a target for the lectin, either alone or in combination with the outer arm linkages. The core linked fucose is the major disease indicator on a number of serum biomarkers for cancer and specific detection of alpha 1-6 as opposed to outerarm linkages provides a significant biomarker. The N224Q mutant is more selective for the 1-6 linkage than outerarm. This offers a significant advantage over the prior art. Consequently, AAL proteins of the present invention have a high affinity to core alpha 1-6 L-fucopyranosyl linkage found in serum protein biomarkers in patient with diseases such as, but not limited to, cancer.

AAL is a 312 amino acid protein which contains five binding sites for L-fucose or L-fucose-linked oligosaccharides. The multivalent nature of AAL gives it an unusually high binding affinity (micromolar) for carbohydrate ligands compared to other lectins. Commercial production of AAL isolates and purifies the lectin by binding to a fucose-starch column. AAL is eluted from the column with 50 mM L-fucose (Fujihashi et al.). Structural and biochemical analysis has shown that commercial AAL has 3 to 5 of its 5 ligand binding sites occupied with fucose as a result of this manufacturing process ( Olausson et al., Amano et al., Fujihashi et al., and Wimmerova et al.).

Surprisingly, it was found that recombinant (r)AAL produced in and isolated from bacteria using nickel affinity chromatography had substantially higher binding affinities for fucosylated oligosaccharides than commercially prepared AAL as determined by surface plasmon resonance studies, tryptophan fluorescence studies and enzyme linked lectin assays.

One embodiment of the present invention is to provide methods for the creation and production of high affinity (recombinant) rAAL and/or mutated rAAL that makes all ligand binding sites available for binding to specific L-fucopyranosyl linkages found in alpha-gal IgG0 and in serum protein biomarkers markers of disease and cancer.

Another embodiment of the present invention is to incorporate the high affinity rAAL as a detector molecule in sensitive and specific blood based assays to determine those at risk for disease such as, but not limited to, cancer.

### Producing and Selecting an High Affinity mutagenic AAL

Random mutagenesis of AAL is obtained by transforming the AAL-pUC57 plasmid into the XL1 Red mutator *E*. *coli* strain (Strategene) following the manufacturers protocol.

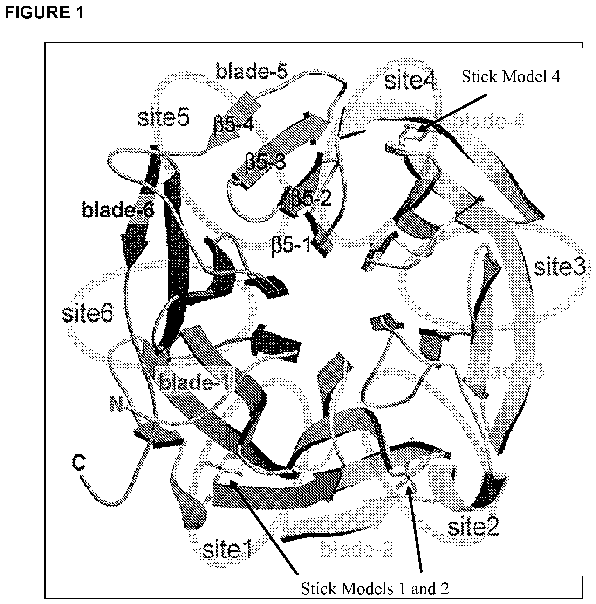

Site directed mutagenesis is focused upon changes in the ligand binding affinities of several of the 5 fucose binding sites in AAL. A *beta*-propeller motif is iterated six times in AAL. This motif is widely conserved in lectins and constitutes the ligand binding domain. The five (5) sites for fucose binding within the motif (see Figure 1) have been described with each site having a different affinity for fucosylated ligands.

As shown in Figure 1, sites 2 and 4 are high affinity ligand binding sites, while sites 1, 3, and 5 have weaker binding affinities. Each *beta* fold contains highly conserved residues specifically involved in fucose binding. In particular sites 2 and 4 have a conserved glutamine residue at positions Q22 and Q75, respectively, which keeps the fucose binding site in an open fucose accessible state. The corresponding residues in sites 3 and 5 are asparagines N129 and N224. Because these asparagine residues (N) are one carbon shorter than glutamine(Q) they do not make the necessary hydrogen bond contacts with adjacent conserved residues to keep these sites in an energetically stable open configuration. A mutant AAL molecule was developed that change the N129 and N224 residues to glutamines (N129Q, N224Q) individually, AALN129Q and AALN224Q and in combination AALN129Q,N224Q to make these sites structurally resemble the high affinity binding sites.

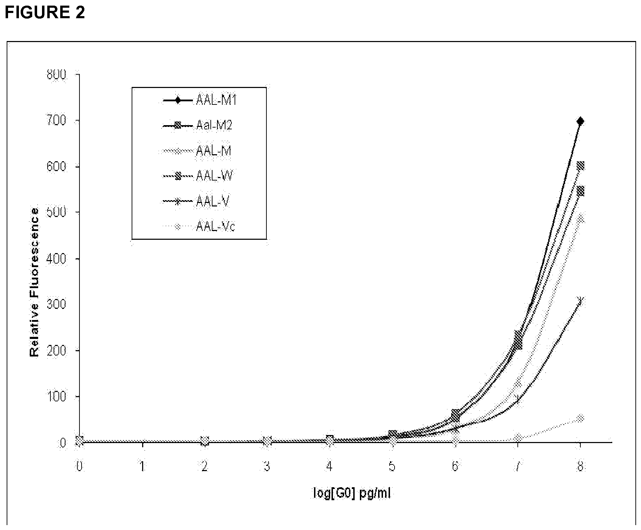

Competition experiments show that these proteins have higher affinity for IgG0 ligands as compared to the wild-type protein (see Figure 2). These results indicate that the random mutagenesis approach will identify specific high affinity ligand binders to the L-fucopyranosyl linkages used for mutant selection. Further described is the development of a mutant library of lectins for selection of other sugar structures beyond those specifically mentioned herein..

A yeast (*Saccharomyces cerevisiae*) based display and selection system is used to screen and select for mutant AAL proteins reactive to specific L-fucopyranosyl linkages. Briefly, a pool of 200-400 XL1 red colonies transformed with AAL-pUC57 and put through the mutator protocol (Strategene) is picked and plasmid DNA prepared. Using standard molecular biology techniques the mutated AAL sequences are subcloned into a version of the yeast display vector pCT302. This system and vector are described in detail in Broder and Wittrup and(2000) Methods Enzymology. Vol. 328, 430-444. Yeast display plasmids containing the mutant AAL library are transformed into a haploid yeast strain EBY100 and grown as described. Mutant AAL proteins are inducibly expressed and displayed on the cell surface of the yeast. The yeast display mutant AAL library is enriched for binding to biotinylated ligand comprising specific L-fucopyranosyl linkages under selection conditions for high affinity binding (Bergan, L., J.A. Gross, B. Nevin, N. Urban, N. Scholler. Cancer Lett 255, 263-74 (2007)). Cells bound to ligand are put through 2 rounds of streptavidin magnetic bead enrichment (Miltenyi Macs) followed by three rounds of flow sorting (Scholler et al., Feldhaus et al., Broder and Wittrup). This system has been shown to obviate expression biases and growth selections common in phage display libraries, and consistently produces rapid enrichment, 10⁵, of selected clones (Feldhaus et al.).

The display library is converted into a secreted mutant AAL library as described by Scholler et al. Mutant AAL DNA is isolated from the enriched display library and cloned by gap repair into the pTOR vector to allow secretion of the mutant AAL proteins into the culture media. Features of the pTOR vector include; an alpha prepro leader to direct secretion of the recombinant protein and in frame additions of a 6X His-tag and an Avitag sequence at the C-terminal end of the mutant proteins (Scholler et al). The Avitag is a biotin acceptor site and is biotinylated at a specific lysine residue when haploid cells transformed with the mutant AAL pTOR library are mated to opposite mating type haploid cells expressing the gene for *E*. *coli* biotin ligase (BirA). Diploid cells are selected for on solid media and grown in liquid culture under inducing conditions for secretion of the His-tagged, biotinylated mutant AAL proteins. Diploid cell culture supernatants are desalted, concentrated and buffer adjusted for Nickel-agarose column purification of AAL-His as per manufacturers protocols (His-Trap, Amersham). Recombinant His-tagged, biotinylated mutant AAL can then be purified and used in subsequent ligand binding studies.

Quantitative equilibrium binding of mutant and wild-type AAL for the selection ligand and other L-fucopyranosyl oligosaccharides are determined by BIAcore analysis and enzyme linked lectin assays (Olausson et al).

### High Affinity Recognition of IgG0 Ligand by Mutant AAL proteins

Equal amounts of recombinant AAL (wild type or mutants) containing 10X Histidine tags were coated to nickel NTA plates and incubated with increasing amounts of IgG0 ligand (X axis). Untagged, commercially purchased AAL (AAL-V) was also coated to nickel NTA plates or on high affinity binding ELISA plates (AAL-Vc) and incubated with increasing amounts of IgG0 ligand. Plates were washed and incubated with fluorescently tagged anti-human IgG, washed and read in a fluorescence detector. Increasing relative fluorescence indicates higher binding affinity. The results showed that the mutant AAL_{M1}(_{N129Q}) exhibited the highest signal to noise ratio, improved the Limit of Detection by 10-100 fold to 10 ng/mL, and increased the dynamic range from 10 ng/mL to 100 ug/mL without saturation (see Figure 2). These results indicate that recombinant mutant AAL proteins will increase the sensitivity and specificity of a blood based assay to detect subtle changes in serum IgG0 levels.

### EXAMPLE 1: rAAL Protein Selective for Cirrhotic Patients.

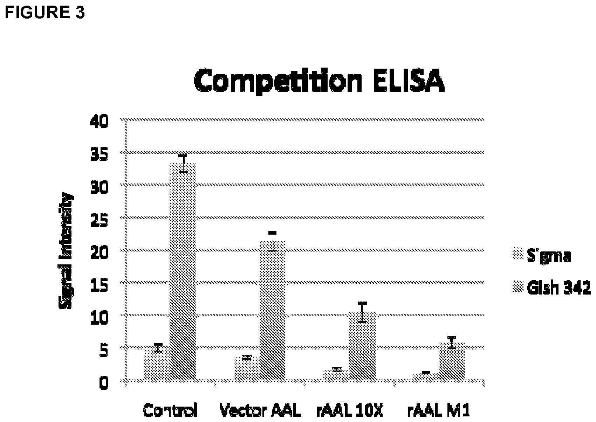

Recombinant lectin (rAAL) was shown to have a higher affinity for the IgG0 ligand than commercially available AAL. In competition ELISA based experiments using serum samples, rAAL had a significantly higher affinity in cirrhotic patients compared with controls (see Figure 3).

### Experimental protocol:

96 well plates are coated with sodium periodate (NalO₄) treated mouse IgG (1.0 ug/well). 3 uL of serum from a patient with cirrhosis (Gish 342) or serum from normal donors (Sigma) were diluted to 100 uL and added to antibody coated wells. Plates are washed and then incubated at room temperature for 30 minutes/shaking with equivalent amounts of either unbiotinylated commercial (Vector) AAL, unbiotinylated recombinant 10X-His tagged (rAAL 10X), or unbiotinylated recombinant 10X-His tagged mutant AAL (rAAL N129Q,N224Q)). Equivalent amounts of biotinylated AAL was then added to each well and incubated for 1 hour at room temperature/shaking. Plates are washed and incubated with streptavidin-800-IRDye as previously described. Plates are washed and fluorescence quantified using a LiCor Odyssey machine. Control samples measured biotinylated AAL binding to IgG0 in the absence of unbiotinylated AAL.

### Experimental results

Competition experiments provide an indirect ELISA for measuring the ability of unbiotinylated commercial (Vector) and unbiotinylated rAAL proteins to interfere with binding of biotinylated AAL to the IgG0 ligand. It is a qualitative indication of the relative affinities of the untagged AAL proteins for the IgG0 ligand.

As shown in Figure 3, rAAL proteins decrease signal intensity to a larger degree than commercially purchased (Vector) AAL in a competition ELISA based format. In serum samples from a cirrhotic patient (Gish 342) unbiotinylated commercial (Vector) AAL will compete with biotinylated AAL and lead to a decrease in signal intensity compared to control (signal output drops from ∼35 to ∼20). Unbiotinylated wild-type rAAL 10X and a mutant rAAL (N129Q,N224Q) protein lead to greater decreases in signal output versus control, indicating a higher affinity for IgG0 in serum from the cirrhotic patient. Sigma= negative control sera (blood sera from individuals with no liver disease).

A further embodiment of the present invention provides for a kit for an assay method according to the invention, said kit comprising a recombinant or mutant form of AAL having a high affinity recognition for specific fucosylated oligosaccharides.

Although the present invention has been described with reference to specific embodiments, workers skilled in the art will recognize that many variations may be made therefrom, for example in the particular selection of a detection molecule linked to AAL herein described, and it is to be understood and appreciated that the disclosures in accordance with the invention show only some preferred embodiments and advantages of the invention. It is to be understood and appreciated that these discoveries in accordance with this invention are only those which are illustrated of the many additional potential applications that may be envisioned by one of ordinary skill in the art, and thus are not in any way intended to be limiting of the invention. Accordingly, other objects and advantages of the invention will be apparent to those skilled in the art from the detailed description together with the claims.

## Claims

1. A method for distinguishing two or more glycoprotein isoforms comprising;
a. obtaining a sample containing glycoprotein;
b. binding said glycoprotein to a recombinant Aleuria aurantia lectin (AAL) containing at least one binding site for L-fucose or L-fucose linked oligosaccharides and having a mutation within at least one of said binding sites, said mutation comprising N129Q and/or N224Q, wherein said lectin has a high affinity for a core alpha 1-6 L-fucopyranosyl linkage in a glycoprotein isoform and wherein said lectin is linked to a reporter molecule; and
c. detecting for the presence of said reporter molecule, the presence of said reporter molecule indicating the presence of the isoform.

2. The method of claim 1 wherein said recombinant AAL is linked in frame to six or ten or more histidine residues.

3. The method of claim 1 wherein said recombinant AAL has ten or more histidine residues linked in frame to the c-terminus.

4. The method of claim 3 wherein said isoform has an alpha-1,6 linked fucose residue.

5. The method of claim 4 wherein said AAL includes mutations in at least one of the sites from a group consisting of R24A, E36A, R77A in domain 2, E898A in domain 2, R179A in domain 4, E191A in domain 4, and combinations thereof.

6. A method for analysing a serum sample obtained from a patient containing a fucosylated glycoform of a target protein comprising:
a. binding a recombinant Aleuria aurantia lectin (AAL) containing at least one binding site for L-fucose or L-fucose linked oligosaccharides and having a mutation within at least one of said binding sites, said mutation comprising N129Q and/or N224Q, wherein said lectin has a high affinity for a core alpha 1-6 L-fucopyranosyl linkage in a glycoprotein isoform, to the target protein having the fucosylated glycoform;
b. detecting the presence of the fucosylated glycoform wherein a change in the amount of fucosylated glycoform present is indicative of the presence of disease.

7. The method of claim 6 wherein said disease is cancer.

8. The method of claim 6 wherein said recombinant AAL is linked in frame to six or ten or more histidine residues.

9. A method for producing a recombinant Aleuria aurantia lectin (AAL) having a high affinity for fucosylated proteins, comprising:
a. obtaining an Aleuria aurantia (AAL) lectin containing at least one binding site for L-fucose or L-fucose-linked oligosaccharides;
b. mutating the lectin within at least one said binding site, said mutation comprising N129Q and/or N224Q; and
c. enriching for said mutated lectin, wherein said lectin has an increased binding affinity for fucosylated proteins;
wherein said increased binding affinity is for the core L-fucosylated linkages on said fucosylated proteins.

10. The method of claim 9 wherein said mutating is by random mutagenesis.

11. The method of claim 9 wherein said mutating is by site directed mutagenesis.

12. The method of claim 9 wherein said enriching is by nickel chromatography.

13. The method of claim 9 whereby said AAL is further linked to six or ten or more histidine residues.

14. The method of claim 9 whereby said AAL is further linked to a reporter molecule.

15. A recombinant Aleuria aurantia lectin (AAL) containing at least one binding site for L-fucose or L-fucose-linked oligosaccharides and having a mutation within at least one of said binding sites, said mutation comprising N129Q and/or N224Q, wherein said lectin has a high affinity for a core alpha 1-6 L-fucopyranosyl linkage in a glycoprotein isoform.

16. The recombinant AAL according to claim 17 wherein said recombinant AAL is linked in frame to six or ten or more histidine residues.

## Patentansprüche

1. Verfahren zum Unterscheiden zwischen zwei oder mehr Glycoprotein-Isoformen, Folgendes umfassend;
a. Erlangen einer Glycoprotein enthaltenden Probe;
b. binden des Glycoproteins an ein rekombinantes Aleuria-aurantia-Lektin (AAL), enthaltend wenigstens eine Bindungsstelle für L-Fucose oder L-Fucose-gebundene Oligosaccharide und eine Mutation an wenigstens einer der Bindungsstellen aufweisend, wobei die Mutation N129Q und/oder N224Q umfasst, wobei das Lektin eine hohe Affinität für eine Kern-Alpha-1-6-L-Fucopyranosylbindung in einer Glycoprotein-Isoform aufweist und wobei das Lektin an ein Reportermolekül gebunden ist; und
c. untersuchen auf das Vorliegen des Reportermoleküls, wobei das Vorliegen des Reportermoleküls auf das Vorliegen der Isoform hinweist.

2. Verfahren nach Anspruch 1, wobei das rekombinante AAL im Leserahmen an sechs oder zehn weitere Histidinreste gebunden ist.

3. Verfahren nach Anspruch 1, wobei das rekombinante AAL zehn oder mehr Histidinreste aufweist, die im Leserahmen an das c-Ende gebunden sind.

4. Verfahren nach Anspruch 3, wobei die Isoform einen Alpha-1,6-gebundenen Fucoserest aufweist.

5. Verfahren nach Anspruch 4, wobei das AAL Mutationen an wenigstens einer der Stellen aus einer Gruppe bestehend aus R24A, E36A, R77A in Domäne 2, E898A in Domäne 2, R179A in Domäne 4, E191A in Domäne 4 und Kombinationen daraus aufweist.

6. Verfahren zum Analysieren einer von einem Patienten erlangten Serumprobe, die eine fucosylierte Glycoform eines Zielproteins enthält, das Folgendes umfasst:
a. Binden eines rekombinanten Aleuria-aurantia-Lektins (AAL), das wenigstens eine Bindungsstelle für L-Fucose oder mit L-Fucose gebundenen Oligosacchariden enthält und eine Mutation an wenigstens einer der Bindungsstellen aufweist, wobei die Mutation N129Q und/oder N224Q umfasst, wobei das Lektin eine hohe Affinität für eine Kern-Alpha-1-6-L-Fucopyranosylbindung in einer Glycoprotein-Isoform an das Zielprotein mit der fucosylierten Glycoform aufweist;
b. erfassen des Vorliegens der fucosylierten Glycoform, wobei eine Änderung der Menge von vorliegender fucosylierter Glycoform auf das Vorliegen einer Erkrankung hinweist.

7. Verfahren nach Anspruch 6, wobei die Erkrankung Krebs ist.

8. Verfahren nach Anspruch 6, wobei das rekombinante AAL im Leserahmen an sechs oder zehn oder mehr Histidinreste gebunden ist.

9. Verfahren zum Erzeugen eines rekombinanten Aleuria-aurantia-Lektins (AAL) mit einer hohen Affinität für fucosylierte Proteine, Folgendes umfassend:
a. Erlangen eines Aleuria-aurantia-(AAL-)Lektins, enthaltend wenigstens eine Bindungsstelle für L-Fucose oder L-Fucose-gebundene Oligosaccaride;
b. mutieren des Lektins an wenigstens einer dieser Bindungsstellen, wobei die Mutation N129Q und/oder N224Q umfasst; und
c. anreichern des mutierten Lektins, wobei das Lektin eine erhöhte Bindungsaffinität für fucosylierte Proteine aufweist;
wobei die erhöhte Bindungsaffinität für die Kern-L-fucosylierten Bindungen an den fucosylierten Proteinen gilt.

10. Verfahren nach Anspruch 9, wobei das Mutieren durch zufällige Mutagenese erfolgt.

11. Verfahren nach Anspruch 9, wobei das Mutieren durch ortsspezifische Mutagenese erfolgt.

12. Verfahren nach Anspruch 9, wobei das Anreichern durch Nickelchromatographie erfolgt.

13. Verfahren nach Anspruch 9, wobei das AAL ferner an sechs oder zehn oder mehr Histidinreste gebunden ist.

14. Verfahren nach Anspruch 9, wobei das AAL ferner an ein Reportermolekül gebunden ist.

15. Rekombinantes Aleuria-aurantia-Lektin (AAL), das wenigstens eine Bindungsstelle für L-Fucose oder mit L-Fucose gebundenen Oligosacchariden enthält und eine Mutation an wenigstens einer der Bindungsstellen aufweist, wobei die Mutation N129Q und/oder N224Q umfasst, wobei das Lektin eine hohe Affinität für eine Kern-Alpha-1-6-L-Fucopyranosylbindung in einer Glycoprotein-Isoform aufweist.

16. Rekombinantes AAL nach Anspruch 17, wobei das rekombinante AAL im Leserahmen an sechs oder zehn oder mehr Histidinreste gebunden ist.

## Revendications

1. Méthode pour distinguer deux ou plus isoformes de glycoprotéines comprenant :
a. L'obtention d'un échantillon contenant une glycoprotéine ;
b. La fixation de ladite glycoprotéine à une lectine recombinante Aleuria aurantia (AAL) contenant au moins un site de liaison au L-fucose ou à des oligosaccharides liés au L-fucose et ayant une mutation dans au moins un desdits sites de liaisons, ladite mutation comprenant N129Q et/ou N224Q, dans laquelle ladite lectine possède une forte affinité au noyau alpha 1-6 L-fucopyranosyle dans un isoforme de glycoprotéine, dans laquelle ladite lectine est liée à un marqueur moléculaire ; et
c. La détection de la présence dudit marqueur moléculaire, la présence dudit marqueur moléculaire indiquant la présence de l'isoforme.

2. Méthode selon la revendication 1 dans laquelle ladite AAL recombinante est liée en cadre à six à dix ou plus résidus d'histidine.

3. Méthode selon la revendication 1 dans laquelle ladite AAL recombinante possède dix ou plus résidus d'histidine liés en cadre à l'extrémité C-terminale.

4. Méthode selon la revendication 1 dans laquelle ledit isoforme possède un résidu de fucose lié en alpha-1,6

5. Méthode selon la revendication 4 dans laquelle ladite AAL comprend des mutations sur au moins un des sites parmi le groupe consistant. R24A, E36A, R77A dans le domaine 2, E898A dans le domaine 2, R179A dans le domaine 4, E191A dans le domaine 4 et des combinaisons de ceux-ci.

6. Méthode pour analyser un échantillon de sérum obtenu chez un patient et contenant un glycoforme fucosylé d'une protéine cible comprenant :
a. La fixation d'une lectine recombinante Aleuria aurantia (AAL) contenant au moins un site de liaison au L-fucose ou à des oligosaccharides liés au L-fucose et ayant une mutation dans au moins l'un desdits sites de liaisons, ladite mutation comprenant N129Q et/ou N224Q, dans laquelle ladite lectine possède une forte affinité au noyau alpha 1-6 L-fucopyranosyle dans une isoforme de glycoprotéine, à la protéine cible ayant le glycoforme fucosylée ;
b. La détection de la présence de la glycoforme fucosylée dans laquelle un changement dans la quantité de glycoforme fucosylée présente est une indication de la présence d'une maladie.

7. Méthode selon la revendication 6 dans laquelle ladite maladie est un cancer.

8. Méthode selon la revendication 6 dans laquelle ladite lectine recombinante AAL est liée en cadre à six, à dix ou plus résidus d'histidine.

9. Méthode pour produire une lectine recombinante Aleuria aurantia (AAL) ayant une forte affinité pour les protéines fucosylées, comprenant :
a. L'obtention d'une lectine Aleuria aurantia (AAL) contenant au moins un site de liaison au L-fucose ou à des oligosaccharides liés au L-fucose ;
b. La mutation de la lectine dans l'un au moins desdits sites de liaison, ladite mutation comprenant N129Q et/ou N224Q ; et
c. L'enrichissement de ladite lectine mutée, dans laquelle ladite lectine possède une affinité de liaison améliorée pour les protéines fucosylées ;
dans laquelle ladite affinité de liaison améliorée est pour les liaisons de noyau L-fucosylés centrales sur lesdites protéines fucosylées.

10. Méthode de la revendication 9 dans laquelle ladite mutation est effectuée par mutagénèse aléatoire.

11. Méthode de la revendication 9 dans laquelle ladite mutation est effectuée par mutagénèse dirigée.

12. Méthode de la revendication 9 dans laquelle ledit enrichissement est réalisé par une chromatographie au nickel.

13. Méthode de la revendication 9 selon laquelle ladite AAL est de plus liée à six ou dix ou plus résidus d'histidine.

14. Méthode de la revendication 9 selon laquelle ladite AAL est de plus liée à un marqueur moléculaire.

15. Lectine recombinante Aleuria aurantia (AAL) comprenant au moins un site de liaison au L-fucose ou à des oligosaccharides liés au L-fucose et possédant une mutation dans au moins un desdits sites de liaison, ladite mutation comprenant N129Q et/ou N224Q, dans laquelle ladite lectine possède une forte affinité au noyau alpha 1-6 L-fucopyranosyle dans un isoforme de glycoprotéine.

16. Lectine recombinante AAL selon la revendication 15 dans lequel ladite lectine recombinante AAL est liée en cadre à six à dix ou plus résidus d'histidine.
